# EUROPEAN PATENT APPLICATION

(11) **EP 1 429 259 A1**
(43) Date of publication of application: **16.06.2004**
(21) Application number: 02760668.0
(22) Date of filing: 20.08.2002
(51) Int. Cl.: G06F 17/30

(54) **BIOLOGICAL SEQUENCE INFORMATION READING METHOD AND STORING METHOD**

(30) Priority: 21.08.2001 US 313488 P
(71) Applicant: Institute of Medicinal Molecular Design, Inc., Tokyo 113-0033 (JP)
(72) Inventor: FUKUDA, Miki, INST. OF MED. MOLECULAR DESIGN INC., Tokyo 113-0033 (JP); SHIGETAKA, Makoto, I. OF MED. MOLECULAR DESIGN INC, Tokyo 113-0033 (JP); TOMIOKA, Nobuo, INST. OF MED. MOLECULAR DESIGN INC, Tokyo 113-0033 (JP); ITAI, Akiko, INST. OF MED. MOLECULAR DESIGN INC., Tokyo 113-0033 (JP)
(74) Representative: Sternagel, Fleischer, Godemeyer & Partner Patentanwälte
(86) International application number: PCT/JP2002/008368
(87) International publication number: WO 2003/017138

(57) **Abstract**

A method of reading biological sequence information comprising a step of judging similarity between biological sequence information as a target of reading and biological sequence information registered in a user side database, and a step of displaying the former information together with the biological sequence information registered in the user side database which is judged to have a similar sequence, and optionally comprising a step of registering the biological information as a target of reading in the user side database.

## Description

### Technical Field

The present invention relates to a method of reading and storing into a database, of biological sequence information related to genome or protein.

### Background Art

Due to the progress of genome study, proteome study and others, information on genome that is a body of genomic information on organisms and information on proteins that are expressed based on genome are rapidly accumulating. Such biological sequence information is accumulated in server computers in public organizations and companies as databases and is utilized.

As examples of databases collecting information on nucleic acid base sequences, GenBank (NCBI, USA), EMBL nucleotide sequence database (EMBL-EBI, Europe), DDBJ (National Institute of Genetics, Japan) and others are open to the public and utilized. Furthermore, there exists a nucleic acid sequence database developed by a company that conducts genomic analysis as a business. As examples of databases collecting information on amino acid sequences of proteins, SwissProt, TrEMBL (both by Swiss Institute of Bioinformatics), GenPept, RefSeq (both by NCBI, USA), PIR (NBRF, USA), PRF (Protein Research Foundation, Osaka) and others are open to the public and utilized.

As an example of database collecting information on steric structures of proteins, Protein Data Bank (RCSB, USA) is known which contains information on amino acid sequence in addition to the information on the three dimensional coordinates of each atom of the protein. As an example of database collecting information on diseases arising from abnormality of genes, OMIM (NCBI, USA) is known.

Among the aforementioned biological sequence information databases, there are those retaining relational information to entries in other database as data. For example, each entry of the SwissProt database retains IDs of entries in EMBL nucleotide sequence database, PIR database, Protein Data Bank, OMIM database and others, that correspond to the amino acid sequence of said entry, as relational information. Moreover, it retains IDs in the PubMed (NCBI, USA) database for literatures reporting basic data regarding said entry. By using such relational information, it is possible to display link information for displaying an entry of other related database by a URL, when a certain entry of SwissProt is displayed on the World Wide Web (WWW) browser for example, so that a user can read the entry of other database easily.

Generally, most of the biological sequence information databases are made public through WWW server, and a user can use from a terminal such as a personal computer through a communication line such as the internet or local area network. On the user side terminal, it is a general practice to search databases and read information obtained by the search using a WWW browser such as the Internet Explorer or the Netscape Navigator. Furthermore, there is a system like GCG Wisconsin Package (Accekrys, USA) wherein the user searches biological sequence information database and read the information from a character display terminal.

As examples of search methods for a biological sequence information database, there are methods such as BLAST (Altschul S. F. et al., J. Mol. Biol. vol.215, pp.403-410, 1990) and FASTA (Pearson W. R. and Lipman D. J., Proc. Natl. Acad. Sci. USA, vol.85, pp.2444-2448, 1988) wherein the search is carried out based on the identity or similarity of nucleic acid base sequences or amino acid sequences, as well as a general search method based on the match or partial match of a keyword in the database. Furthermore, "sequence alignment method" is also used frequently, which searches the correspondence between sequences for multiple data with similar sequences. Examples of the sequence alignment method include Smith-Waterman algorithm (Smith T. F. and Waterman M. S., J. Mol. Biol., vol.147, pp.195-197, 1981) and Clustal-W (Thompson J. D. et al., Nucleic Acids Res., vol.22, pp.4673-4680, 1994).

### Disclosure of Invention

As a method of reading the information that a user has obtained by search using the aforementioned biological sequence information database, a method of displaying the information on the screen of a WWW browser or a character display terminal is common. Furthermore, as a method of storing the aforementioned information for later use, a method of storing text data as a file on the terminal using the data storing function of the WWW browser or the character display terminal is common. However, by these methods, the data to be read or stored are treated independently in units of files obtained by every search trial, and consequently, various problems as mentioned below will arise. When a user reads biological sequence information data including nucleic acid base sequences or protein amino acid sequences, the user often wants to compare between data with high sequence homology or between data of nucleic acid base sequence and its corresponding protein amino acid sequence. Since the relation between data is lost with the above-mentioned methods of reading/storing the data in units of files, it is inconvenient for the user that he/she needs to manage the relation between files by himself/herself, with a method of recording the relation separately for example.

When a user conducts sequence alignment for multiple sequence data, the user needs to read/store data files from which the respective sequence data are derived and the result file of the sequence alignment separately, by the methods of reading/storing in units of files. Also in this case, the user often wants to examine respective files by comparing them, or to repeat the sequence alignment by altering/adding calculation parameters or some of the sequence data, and therefore, the conventional methods are inconvenient.

The present invention was completed for the purpose of solving the above-mentioned problems and allowing the user to manage and easily read the information obtained by search of biological sequence information databases on the user side terminal such as a personal computer. To be more specific, the object of the present invention is to allow a user to read biological sequence information obtained on the terminal such as the entry of the biological sequence information database or the result of sequence alignment while referring to the user side database, and further, to store the information in the user side database, and to manage and read said information easily.

As a result of zealous endeavor, the inventors found out that the aforementioned object can be solved by extracting sequence information, sequence alignment information, steric structure information, or annotation information from the biological sequence information data obtained on the terminal and by storing the information in a database.

By the present invention, there is provided a method of reading biological sequence information comprising:
(a) a step of designating one or more biological sequence information to be a target of reading as a reading sequence,
(b) a step of judging similarity between the reading sequence and one or more biological sequence information registered in a user side database, and
(c) when the reading sequence is similar to one or more biological sequence information in the user side database, a step of displaying the biological sequence information in the user side database as a similar sequence together with the reading sequence.

According to preferred embodiments of the present invention, the following methods are provided.

The aforementioned method, wherein the reading sequence is designated in step (a) based on the information displayed on the terminal;

The aforementioned method that uses documents in HTML format, XHTML format, or XML format as the information displayed on the terminal;

The aforementioned method, wherein the judgment of similarity in step (b) is carried out with a sequence alignment method;

The aforementioned method, wherein the judgment of similarity is carried out by the method described in the PCT International Publication WO 01/13268 ("EigenID");

The aforementioned method, wherein a sequence alignment between the reading sequence and the similar sequence is displayed in step (c);

The aforementioned method, wherein the relation between the reading sequence and the similar sequence is displayed as a group or in a hierarchy in step (c);

The aforementioned method, wherein respective annotation information on the reading sequence and the similar sequence are merged and displayed in step (c);

The aforementioned method, wherein a steric structure of the protein corresponding to either one or both of the reading sequence and the similar sequence is displayed;

One of the aforementioned methods, which further comprises the following step: (d) a step of storing the reading sequence in the user side database;

The aforementioned method, wherein the reading sequence and the similar sequence are stored as a group or in a hierarchy in step (d);

The aforementioned method, wherein respective annotation information on the reading sequence and the similar sequence are merged and stored in step (d);

The aforementioned method, wherein the sequence alignment between the reading sequence and the similar sequence is stored in step (d);

The aforementioned method, wherein the storing of the sequence alignment is carried out with the method described in the PCT International Publication WO 00/43939;

One of the aforementioned methods, which further comprises the following step: (e) a step of obtaining information from the server computer using a data item in the user side database as a query and setting the information as the reading sequence in step (a);

The aforementioned methods which comprise a step of generating an information source specification that corresponds to a data item in the user side database using an auxiliary database storing the information sources for respective kinds of data items; and

A method of obtaining information from the server computer using a data item in the user side database generated by one of the aforementioned methods as a query;

A method of highlighting a corresponding item in the document being read on the terminal, when said item is found to be corresponded to a data item in the user side database, using the user side database generated by one of the aforementioned methods;

A computer-readable media recording a program to carry out the aforementioned methods; and

A computer-readable media recording the user side database generated by the aforementioned methods.

Furthermore, by the present invention, there is provided a method of reading biological information data by extracting one or more information selected from a group comprising sequence information, sequence alignment information, steric structure information, and annotation information from the biological sequence information data and storing the information in a database, which is characterized by:
(a) that storing in the database is carried out at the terminal side connected to the server computer via a communication means, and
(b) that the information in the database is grouped based on the identity or homology of the sequence information.

In preferred embodiments of the present invention, there are provided,

The aforementioned method wherein the information in the database is grouped based on the sequence alignment information;

The aforementioned method, characterized by merging, upon the grouping, steric structure information and/or annotation information between the data whose sequences are judged to be similar;

The aforementioned method, wherein, upon the grouping, steric structure information and/or annotation information is copied or linked between the data whose sequences are judged to be similar;

The aforementioned method, wherein the grouping is carried out based on multiple criteria;

The aforementioned method, wherein the grouping is carried out accompanying a hierarchical structure;

The aforementioned method wherein the judgment of similarity of the sequence information is carried out by the method described in the PCT International Publication WO 01/13268 ("Eigen ID");

The aforementioned method, wherein an alignment between similar sequences are stored;

The aforementioned method, wherein the storing of the alignment is carried out by the method described in the PCT International Publication WO 00/43939;

A method of obtaining information from the server computer and registering in the database using a data item in the database generated by the aforementioned method as a query;

A method of generating an information source specification that corresponds to a data item in the database generated by the aforementioned method using an auxiliary database storing the information sources for respective kinds of data items; and

A method of highlighting a corresponding item in the document being read on the terminal, when said item is found to be corresponded to a data item in the database generated by the aforementioned method.

### Brief Explanation of Drawings

Fig. 1 is a flow chart of a preferred embodiment of the method of the present invention.
Fig. 2 shows an example of designating the data in the SwissProt database as the reading sequence of the present invention.
Fig. 3 is shows an example of a procedure of determining the similarity between the reading sequence and biological sequence information in the user side database and selecting the similar sequence.
Fig. 4 shows an example of screen-display of the program KeyMine.
Fig. 5 shows an example of displaying the sequence alignment together with the steric structure of the protein by the program KeyMine.
Fig. 6 shows an example of an auxiliary database storing a template of URL.

### Best Mode for Carrying out the Invention

Meanings and definitions of the terms in the present description are as follows.

"Sequence information" is a concept including information on nucleic acid base sequence or information on amino acid sequence of a protein. Sequence information is usually represented by the kind of nucleic acid base or amino acid residue with one-letter or three-letter codes (for example, an amino acid residue alanine is represented by an one-letter code "A" or by a three-letter code "ALA"), and by arranging these codes in the order of the sequence.

"Biological sequence information" is a concept comprising information on nucleic acid base sequence and its partial sequence related to organisms including genome / cDNA / mRNA / EST (expressed sequence tag) / SNP (single nucleotide polymorphism) / DNA fragment / RNA fragment, and information on amino acid sequence and its partial sequence related to organisms including protein / protein domain / peptide fragment / physiologically active peptide, and may contain one or more kinds of annotation information in addition to the sequence information (when two or more terms are concatenated with a "/" in the present description, "/" means "and/or" unless otherwise specified).

"Partial sequence" is a continuous sequence which is a part of a certain sequence.

"Annotation information" is the information that is stored in addition to the sequence information in the database or file of the biological sequence information, and any form is acceptable. Examples of annotation information include information on the function / expressing site in an organism / sequence homology for a gene or a protein, information on the characteristics / modification / mutation / function for a specific site / specific region of a sequence, information on the sequence alignment, information on the steric structure of a protein, information on the compound interacting or binding to a protein, information on the literature / information source from which said biological sequence information is derived, information on the relation (link information) to a data item in the same database or other databases, and the like.

"Server computer" is a computer that accumulates information including biological sequence information as a database or a file and provides services such as registration, search, analysis, and display of the data to a user.

"Terminal" is a user side device that exchanges information via a communication means with the server computer and displays it, and includes computers such as a character display terminal which only treats character information and a personal computer running a WWW browser to treat WWW server information.

"WWW server" is a server computer that can transmit information to a terminal in HTML (hyper text markup language) format, XHTML (extensible HTML) format, or XML (extensible markup language) format. The specifications of the HTML format, XHTML format, and XML format are available on the web site of the World Wide Web Consortium (http://www.w3.org).

"WWW browser" is a software used on the terminal to display characters / figures / images based on the information received from the WWW server in HTML format, XHTML format, or XML format. Examples of WWW browser include Internet Explorer (Microsoft Inc.) and Netscape (Netscape Inc.).

"Link information" is information indicating that certain biological sequence information is related to other biological sequence information or information other than the biological sequence information. In the HTML format, XHTML format, and XML format, link information is represented by a syntax (hereinafter referred to as "URL") called URI (uniform resource indicator) or URL (uniform resource locator).

"Sequence similarity" is a concept which describes the degree of similarity between two sequence information, and includes cases wherein one sequence is a partial sequence of the other sequence, or two sequences are completely identical. The sequence similarity is usually determined by counting the number of nucleic acid bases or amino acid residues that are judged to be the same or similar between two sequences after making correspondence between two or more sequences by the sequence alignment method, and is expressed as a ratio to the number of all nucleic acid bases or amino acid residues. For determining whether the sequences are exactly identical or not, the method described in the PCT International Publication WO 01/13268 can be used, besides the aforementioned alignment method.

"Sequence alignment" means a procedure of making correspondence between two or more sequences so that nucleic acid bases or amino acid residues match as many as possible, and the correspondence obtained as a result of the procedure.

"Sequence ID" is a short character string of a fixed or variable length added to the biological sequence information for distinguishing it. Examples of sequence IDs include accession numbers of GenBank and SwissProt and identification information of SwissProt.

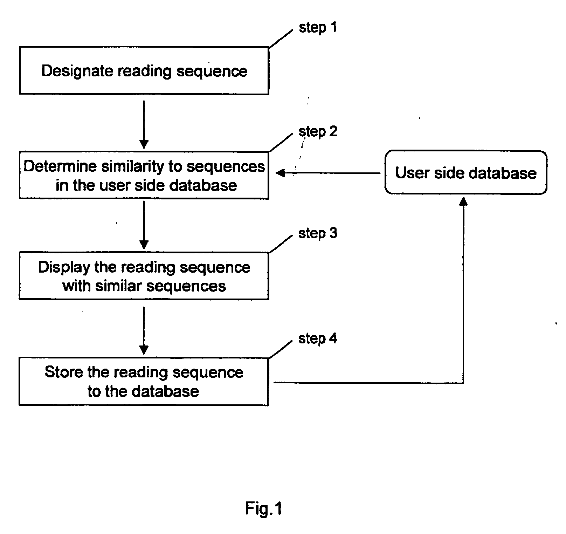

In the following, the method of the present invention is explained concretely with an example of a procedure of reading and storing a piece of biological sequence information (Fig.1). The following procedure merely shows a preferred embodiment of the present invention, and thus, it is needless to say that the scope of the present invention is not limited to the following embodiment.

First, the user designates a piece of biological sequence information as a target of reading (hereafter referred to as the "reading sequence") (step 1). Examples of the methods of designation include a method of designating a file containing the biological sequence information; a method of selecting whole or a part of the document containing biological sequence information displayed on the terminal in text format, HTML format, XHTML format, or XML format; a method of downloading the biological sequence information from a server computer; a method of downloading the related biological sequence information from an appropriate server computer based on the link information such as the URL in the document displayed on the terminal; a method of obtaining from the printed material containing biological sequence information with optical scanning and character recognition methods; and a method wherein the user inputs a character string representing the biological sequence information with a keyboard.

Examples of biological sequence information to be designated in step 1 include nucleic acid base sequence information obtained from the databases such as GenBank, EMBL and DDBJ; protein amino acid sequence information obtained from protein amino acid sequence databases such as SwissProt, TrEMBL, PIR, PRF and GenPept; protein steric structure information and amino acid sequence information obtained from PDB; sequence information obtained by search methods such as FASTA and BLAST to the sequence information databases.

When the biological sequence information designated in step 1 is represented in HTML format, XHTML format, or XML format, it is preferable to remove the markup notation (also called "tag") to take out the body of the biological sequence information. In this case, the tag representing an URL may be regarded as link information and added to the annotation information.

When a search result of FASTA or BLAST is assigned as the biological sequence information in step 1, said search result usually contains only one or more partial sequence corresponded to the query sequence of the search. In this case, said partial sequence may be treated as the sequence information of the reading sequence, but it is also acceptable to obtain complete corresponding sequence information from an appropriate server computer based on the ID or URL of the sequence in said search result, and to treat it as the sequence information of the reading sequence instead of the partial sequence.

Step 1 may be carried out by the user by explicitly designating the reading sequence, but alternatively, it may be carried out automatically in response to a certain trigger. Examples of the trigger include the occasion when a certain time has passed; the occasion when the program carrying out the method of the present invention is activated; the occasion when the information displayed on the terminal is updated; the occasion when the user moves the cursor or the pointer on the terminal onto the displayed text including biological sequence information; and the occasion when the user switches between windows to be operated on the terminal.

When the biological sequence information designated in step 1 contains two or more independent pieces of sequence information, it is preferable to treat each sequence as a reading sequence and carry out the following procedure similarly. Or as an alternative method, it is acceptable to treat such sequence information equivalent to the biological sequence information in the user side database, and to carry out the following procedure.

Next, sequence similarity between the reading sequence and one or more biological sequence information stored in the database is determined, and one or more biological sequence information having sequence information similar to the reading sequence (hereafter referred to as "similar sequence") is selected from the database (step 2). The database used here (hereafter referred to as "user side database") may be in any form as long as the sequence information can be stored, but it is preferable to use those that can store biological sequence information including annotation information. It is more preferable to use those that can store the annotation information separately according to its kind. It is preferable that the user side database is stored in the terminal on the user side, but the database may be stored in the server computer or other computer as long as the user can add / change / obtain the data via a communication means.

Judgment of similarity in step 2 can be carried out by a sequence alignment method between the reading sequence and the sequence information in the user side database. In this case, it is preferable to select a sequence with a similarity beyond a certain value (for example, more than 90%) as a similar sequence. It is acceptable to allow the user to optionally change the threshold of the similarity. Examples of the sequence alignment methods used here include FASTA, BLAST, Smith-Waterman algorithm and CLUSTAL-W.

When the reading sequence is the information on nucleic acid base sequence, the judgment of the sequence similarity may be carried out with the base sequence itself, or alternatively, it is also acceptable to obtain an amino acid sequence of the corresponding protein by translating said base sequence and regarding the amino acid sequence as the sequence information of the reading sequence. Furthermore, when the information on the translated amino acid sequence is registered as annotation information in addition to the nucleic acid base sequence, as in the biological sequence information obtained from EMBL or GenBank, said amino acid sequence may be treated as the sequence information as well.

For the purpose of selecting a sequence that exactly matches the reading sequence as a similar sequence in step 2, a method of ordinary text comparison may be used, however, it is more preferable to use the method described in PCT International Publication WO 01/13268 (hereafter referred to as "EigenID method"). By the EigenID method, it is possible to determine the sequence similarity (exact match) quite rapidly compared with the case by the sequence alignment method. Furthermore, the sequence alignment method and the EigenID method may be used together. For example, the sequence alignment method which relatively takes time is used only for the data items in the user side database where the similarity is expected to be high based on the annotation information and the link information, and similarity (exact match) may be judged using the rapid EigenID method to other data items in the user side database.

For the purpose of selecting a sequence in step 2 that is in relation of a partial sequence to the reading sequence or the reading sequence is in relation of a partial sequence as the similar sequence, it is also acceptable to use a general text comparison method instead of the sequence alignment method.

Usually, step 2 is carried out using the entire reading sequence, but alternatively, it may be carried out using one or more partial sequences of the reading sequence. For example, when the fact that the reading sequence can be divided into two or more functional or structural domains is described in the annotation information, one or more partial sequences obtained by dividing said reading sequence into domains may be treated as the reading sequence in step 2.

At the stage of carrying out step 2, it is preferable that the user side database contains one or more biological sequence information, but when the user employs the method of the present invention for the first time, it is not a problem even if the user side database is empty. In this case, it is treated as if no similar sequence is found in step 2.

Next, one or more similar sequences found in step 2 are displayed together with the reading sequence (step 3). It is preferable to display appropriate annotation information in addition to the sequence information. By step 3, the user can read biological sequence information whose sequence is similar together with the biological sequence information of the reading sequence, so that he/she can deepen the understanding of the reading sequence. When a similar sequence is not found in the aforementioned step, only the biological sequence information of the reading sequence is displayed.

As one of the preferred embodiments of the present invention, a method of displaying sequence alignment between the reading sequence and the similar sequence in step 3 is provided. By displaying the sequence alignments, the user can easily recognize similar / different parts between the reading sequence and the similar sequence. Furthermore, by displaying the sequence alignment together with the annotation information on characteristics / modification / mutation / function of a specific site / specific part of the sequence, the user can deepen the understanding of the reading sequence or the similar sequence. For example, when there is annotation information on function A for a certain site in the similar sequence, it can be presumed that the corresponding part of the reading sequence is possibly related to the function A. For such purposes, it is convenient to display the annotation information on the specific site / specific part of the sequence and the corresponding part of the alignment representation by relating them with the same colors and marks.

As one of the preferred embodiments of the present invention, a method of displaying steric structure(s) together with the sequence in step 3 is provided, when either one or both of the reading sequence and the similar sequence have information on protein steric structure in their annotation information. By viewing the steric structure display together, the user can deepen the understanding of the reading sequence or the similar sequence. Based on the annotation information on characteristics / modification / mutation / function of the specific site / specific part of the sequence, corresponding parts of the protein steric structure may be displayed with coloring and marking.

By displaying the aforementioned sequence alignment and protein steric structure together, the user can further deepen the understanding of the reading sequence or the similar sequence. When two or more of the reading sequence or the similar sequence have information on the protein steric structure in their annotation information, it is preferable to display their steric structures by superposing them based on the sequence alignment. This superposition can be carried out by superposing the positions of alpha carbons or main chain atoms of the amino acid residues corresponded with the sequence alignment by the Kobash's least squares algorithm (Kabsh W, Acta Cryst. Sect.A, vol.32, pp.922-923, 1976). By displaying steric structures with superposition, the user can examine easily similar parts / different parts between the sequences and similar parts / different parts between steric structures.

As one of the other preferred embodiments of the present invention, a method of displaying the reading sequence and the similar sequence with hierarchy or as a group in step 3 is provided. Examples of the methods of displaying with hierarchy include a method of displaying the ID of the similar sequence in a tree subordinate to the ID of the reading sequence, and a method of displaying the ID of the reading sequence in a tree subordinate to the ID of the similar sequence. Examples of the methods of displaying as a group include a method of displaying the ID of the reading sequence along with the ID of the similar sequence. Such hierarchical / grouped displays with the IDs of the sequences are helpful for the user to understand the relation between the displayed biological sequence information easily when information on many reading sequences / similar sequences is displayed at the same time.

As one of the other preferred embodiments of the present invention, there is provided a method of displaying, in step 3, respective annotation information of the reading sequence and the similar sequence after merging them together. For example, when annotation information A is attached to the reading sequence and annotation information B is attached to the similar sequence, respectively, both A and B are merged and displayed as annotation information of the reading sequence or the similar sequence. Displaying annotation information after merging makes it possible to read the annotation information of the reading sequence and the similar sequence at the same time, and helps user to understand the biological sequence information. Merging of annotation information is not always necessary for all annotation items, but may be carried out for some part of the items such as the annotation information on the functions of genes or proteins.

As a further preferred embodiment of the present invention, there is provided a method including a step (step 4) wherein the information on the reading sequence is stored in the user side database, in addition to the aforementioned steps 1 to 3. In this case, it is preferable to store the annotation information in addition to the sequence information of the reading sequence. With step 4, information on the reading sequence that was once read by the user is stored in the database, and can be treated as an object for reading and searching a similar sequence afterwards. As the user employs the method of the present invention repeatedly, biological sequence information that the user has read is accumulated in the user side database. Thus, the problem of information management after reading which exists in the conventional reading method of the biological sequence information, is solved.

In step 4, it is preferable to store the reading sequence and the similar sequence as a group or with hierarchy. Examples of the methods of grouping include a method of storing the IDs of sequences that are similar as a listed table in the database; and a method of storing the IDs of sequences that are similar for each biological sequence information. Here, "sequences that are similar" means one or more biological sequence information consisting of similar sequences corresponding to the reading sequence. Examples of the methods of storing with hierarchy include a method of storing in the database a listed table of correspondence from ID of the similar sequence to the ID of the reading sequence; and a method of storing in the database a listed table of correspondence from the ID of the reading sequence to the ID of the similar sequence. Storing the similar sequence as a group or with hierarchy makes it possible to display sequences that are similar as a group or with hierarchy when the biological sequence information contained in the user side database is read.

When the output of the similar sequence search methods such as FASTA and BLAST is designated as the reading sequence in step 1, sequences judged to be similar in said output may be stored as a group or with hierarchy. For example, treating the query sequence of the similar sequence search as the reading sequence, and treating the obtained similar sequence by the search as the similar sequence, and they may be stored in the user side database as a group or with hierarchy similarly by the aforementioned method.

The aforementioned method of grouping and making hierarchy is not particularly limited to one kind, and for example, it is acceptable to store similar sequences selected by a certain similarity threshold by a sequence alignment method and similar sequences selected by a different similarity threshold as separate groups. As another example, it is acceptable to store similar sequences selected by the sequence alignment method and similar sequences selected by the EigenID method (sequences matching exactly) as separate groups. Moreover, it is also acceptable for the user to designate two or more arbitrary biological sequence information and to store them as a group or with hierarchy.

In step 4, it is acceptable to merge respective annotation information between the reading sequence and the similar sequence and store them. For example, when annotation information A is attached to the reading sequence and annotation information B is attached to the similar sequence respectively, it is acceptable to store in the database both A and B as the annotation information of the reading sequence. Furthermore, it is acceptable to carry out the merging similarly for the annotation information of the similar sequence and to update the database. Merging of the annotation information is not necessarily carried out for all items of the annotation information, and it is acceptable to carry out the merging to certain items limitedly such as the annotation information on the functions of genes or proteins for example.

In step 4, it is acceptable to store the sequence alignment between the reading sequence and the similar sequence as the annotation information of the reading sequence and/or the similar sequence. For example, when the sequence alignment method is used in the judgment of sequence similarity in step 2, it is preferable to store the obtained sequence alignment as annotation information. Sequence alignment may be stored as a text data representing it, but preferably, it is recommended to use the method described in PCT International Publication WO 00/43939. By this method, it is possible to store sequence alignment in a compressed form and expand it easily at the time of reading.

When the output of the similar sequence search method such as FASTA and BLAST is designated as the reading sequence in step 1, the alignment of partial sequences in the output may be stored as annotation information. When complete sequence information corresponding to the partial sequence in the output of FASTA or BLAST is obtained from the server computer and treated as the reading sequence, it is recommended to store the correspondence between the partial sequence in the sequence alignment and the complete sequence information together with the sequence alignment.

When information on the protein steric structure is stored as the annotation information in step 4, it is recommended to store the correspondence between the . steric structure and the sequence information of the reading sequence as annotation information. When data from PDB are designated as the reading sequence, for example, there are cases in which some part of the amino acid residues are missing in the steric structure information described in the ATOM records compared to the sequence information described in the SEQRES records. In such cases, it is convenient for displaying the steric structure in step 3, to store the range of the amino acid sequence where the steric structure exists.

In step 4, it is acceptable for the user to add arbitrary,annotation information to the reading sequence or to the similar sequence and store them. Examples of the annotation information to be entered include user's review of the reading sequence or the similar sequence and experimental data. Examples of other embodiments of the addition of annotation information include date and time when the reading sequence is input; URL of the data source of the reading sequence; method of judging the similarity used in step 2, which may be automatically generated by the program and stored as annotation information.

As one of the other embodiments of the present invention, there is provided a method of highlighting a data item in the document being read on the terminal by coloring or marking, when said item is found to be identical or related to one of the data items in the user side database, based on the data items in the user side database. Document being read in this case is not limited to that of the biological sequence information, rather any type of document is acceptable as long as it can be displayed on the terminal. As an example, a word is extracted from the document being read, existence of said word is judged by text search to the items of the annotation information in the user side database, and if said word is found in the annotation information in the user side database, the corresponding part of the document being read can be highlighted. Together with the highlighting, it is more convenient to display the corresponding items in the user side database.

As further different embodiment of the present invention, there is provided a method of obtaining biological sequence information from a WWW server using a data item in the user side database as a query. For example, a SwissProt ID is extracted from the user side database, search is carried out to the WWW server providing the SwissProt database using said ID as a query, and biological sequence information corresponding to said ID is obtained. Furthermore, by treating the obtained biological sequence information as the reading sequence in step 1, any method of the present invention may be applied. This method makes it possible to read the latest information and update the data items in the user side database for the biological sequence information that are frequently updated. This method can be applied not only to the biological sequence information but also to any information as long as the information can be obtained based on the annotation information stored in the user side database. Examples of such information include PubMed and OMIM (both by NCBI, USA).

To carry out the aforementioned method, it is recommended to prepare and use an auxiliary database that stores the data source for respective types of biological sequence information. This auxiliary database stores templates of URLs describing the data source of the biological sequence information of respective types such as SwissProt, BenBank and PDB, for example. A template of URL is obtained from the auxiliary database depending on the type of the biological sequence information to be obtained, and an URL for obtaining the information from the WWW server is generated based on the template and the ID of the biological sequence information to be obtained. By sending a query to the WWW server using the generated URL, the desired biological sequence information is obtained.

### Examples

The present invention will be explained more specifically with reference to examples. However, the scope of the present invention will not be limited to the following examples.

### Example 1

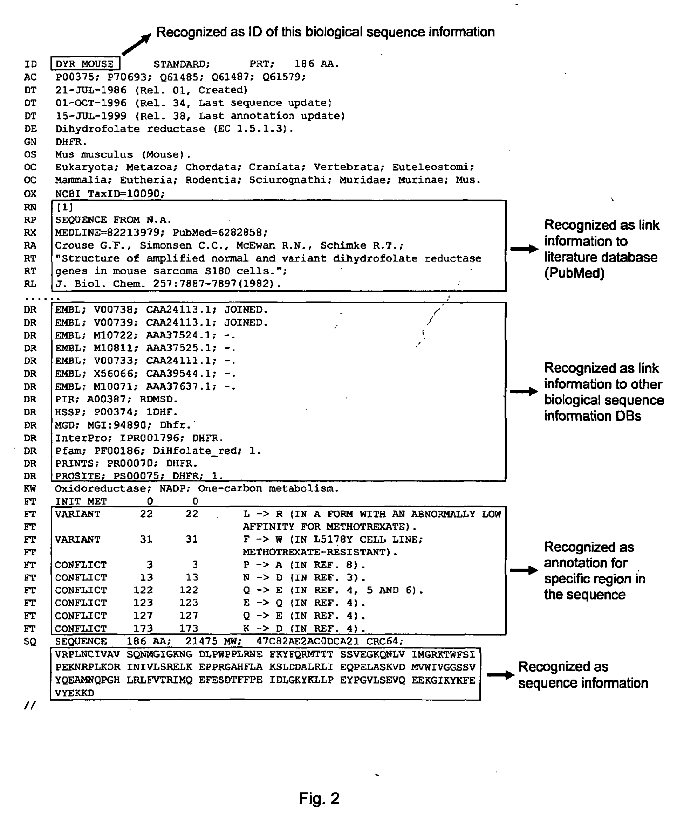

An example of designating the data DYR_MOUSE of the SwissProt database as a reading sequence of the method of the present invention is shown (Fig.2). "DYR_MOUSE" in the first line with a header "ID" is recognized as the ID of this biological sequence information. Alternatively, contents in the line with a header "AC" (accession numbers) may be used as the ID of the sequence (for example "P00375"). Lines between the line with a header "SQ" and the line with a header "//" represent amino acid sequence information, and these lines are recognized as sequence information.

Other lines are treated as annotation information. Lines with a header "FT" represent annotation information on specific sites / specific parts of the sequence, wherein "VARIANT" represents a mutation of a specific site and "CONFLICT" represents a site where conflicting experimental data are known. These annotation information is used in the methods of the present invention for the purpose of coloring or marking specific sites / specific parts of the sequence in the sequence alignment display or in the protein steric structure display.

Lines with a header "DR" represent annotation information on links to other biological sequence information databases, which is used in the methods of the present invention for the purpose of obtaining corresponding biological sequence information from the server computer using the data item in the user side database as a query. For example, "EMBL" is recognized as a link to the EMBL nucleic acid base sequence database, and "V00738" is recognized as an ID (accession number) of the data item.

Lines following a header "RN" represent annotation information on links to the literature information database (PubMed), which is used in the methods of the present invention for the purpose of obtaining corresponding literature information from the server computer using the data item in the user side database as a query. For example, the number 6282858 in "PubMed=6282858" is recognized as an ID of the PubMed.

### Example 2

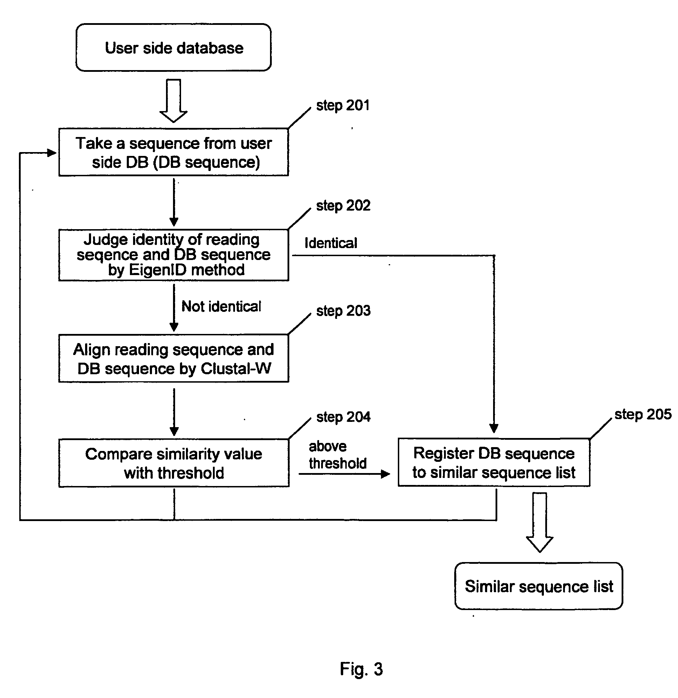

An example of a procedure of determining the similarity between the reading sequence and the biological sequence information in the user side database and selecting a similar sequence is shown in Fig.3.

First, one sequence (referred to as "DB sequence" in this example) is taken from the user side database (step 201). Next, judgment is carried out by the EigenID method whether the reading sequence exactly matches the DB sequence (step 202). This step can be carried out rapidly by using hash. If the reading sequence and the DB sequence are judged to be identical, said DB sequence is added to the list of the similar sequences (step 205). In the list of the similar sequences, an index of the corresponding data item in the user side database is stored. The list of the similar sequences has been made empty when a new reading sequence is entered into the procedure of the present example.

If the reading sequence and the DB sequence are judged to be not identical in step 202, sequence alignment is carried out by Clustal-W between the reading sequence and the DB sequence (step 203). Similarity value is calculated between the reading sequence and the DB sequence based on the obtained sequence alignment, and is compared to the pre-determined threshold value (for example 90%) (step 204). When the similarity value is higher than the threshold value, said DB sequence is added to the list of the similar sequences in step 205.

By repeating the aforementioned procedures for all biological sequence information in the user side database, a list of the similar sequences having similar sequence information to the reading sequence can be obtained.

A hash value of the sequence information is calculated and used in the EigenID method used in step 202, and by calculating hash values for the biological sequence information in the user side databases beforehand, step 202 can be carried out more rapidly. If the user wants to treat only the biological sequence information with exactly matching sequence as the similar sequence, it is not necessary to carry out steps 203 and 204.

### Example 3

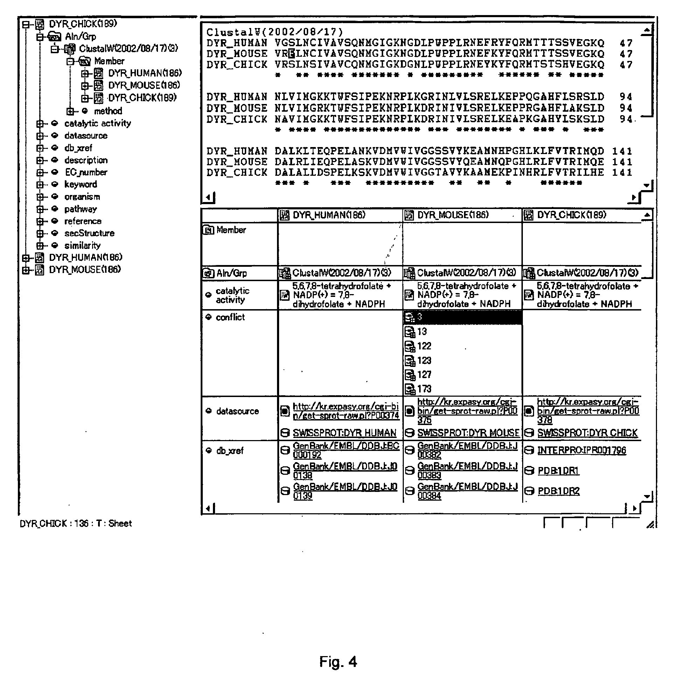

An example of displaying the reading sequence and the similar sequence together is shown in Fig.4.

Fig. 4 is the screen snapshot when amino acid sequence information of dihydrofolate reductase of human (DYR_HUMAN), mouse (DYR_MOUSE), and chicken (DYR_CHICK) is obtained from the SwissProt, and taken into the program KeyMine which carries out the methods of the present invention.

In the lower-right section of the screen, annotation information in the SwissProt for each protein is displayed separately for respective types of annotation. For example, the "conflict" field shows information on the position of amino acid sequence where conflicting experimental data are known and the "db_xref" field shows link information to the data items of other biological sequence information databases. The "datasource" field shows annotation information indicating the data source which is automatically added when KeyMine has imported the reading sequence.

For annotations representing link information to external information source such as "datasource" and "db_xref", the user can obtain corresponding information from external information source by instructing the program by clicking on an item on the display of the annotation information. If the information to be obtained is biological sequence information, it is also possible to treat the information as a reading sequence. If the information to be obtained is general information on the WWW server, WWW browser will be activated and the information is displayed on the screen.

In the left side section of the screen, summary information on the imported biological sequence information is displayed as tree representation (the tree for DYR_CHICK is shown on the screen, and trees for other sequence information are collapsed). Respective nodes below "catalytic activity" in the tree indicate the types of the annotation information. At the node "Aln/Grp" in the tree, summary information on the sequence alignment generated by Clustal-W is displayed. Below the node "Member", IDs of the three sequences (DYR_HUMAN, DYR_MOUSE, DYR_CHICK) are displayed as a group.

In the upper-right section of the screen, sequence alignment generated by Clustal-W is displayed. Display of the annotation information and display of the sequence alignment are mutually interlinked. For example, in Fig.4, by selecting "conflict" annotation of residue 3 of DYR_MOUSE, one-letter code of the corresponding residue in the sequence alignment display is highlighted (in reversed drawing). Furthermore, residues in the sequence alignment display can be color-coded based on the annotation information on the specific site / specific part of the sequences, for example corresponding residue to the "conflict" annotation can be color-coded. (Colors are not shown in Fig.4 for the sake of convenience).

### Example 4

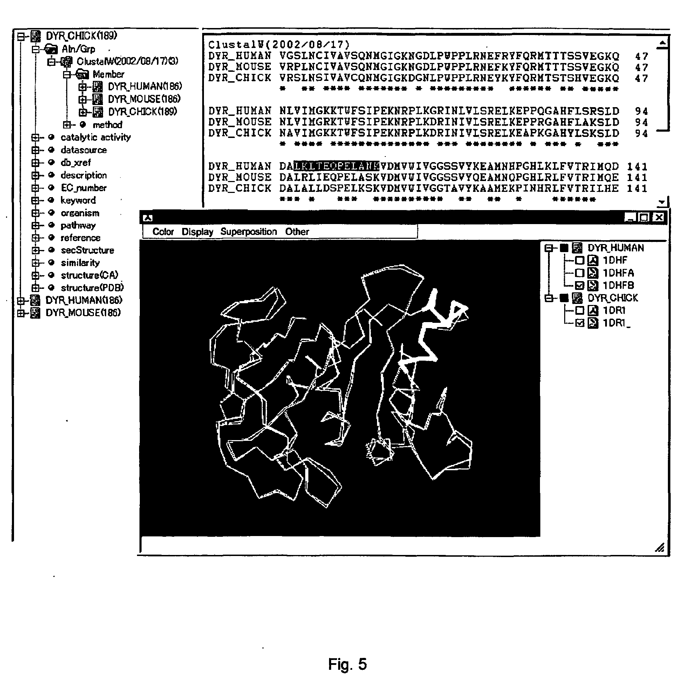

An example of displaying the steric structure information of protein together with the biological sequence information is shown in Fig.5, when it is available as annotation information.

Among the dihydrofolate reductases treated in Example 3, protein steric structures of DYR_HUMAN and DYR_CHICK are known by crystal analysis, and it is possible to obtain data of the crystal structures based on the link information to the PDB in the annotation information of the SwissProt. Fig.5 shows the screen shot when data on the crystal structure of human enzyme (1DHF) and the crystal structure of chicken enzyme (1DR1) are downloaded from the PDB and displayed by KeyMine. In KeyMine, information on protein steric structure downloaded as above can be stored in the user side database as one type of the annotation information.

Steric structures of two kinds of enzymes are displayed superposed in a separate window at the lower part of the screen. This superposition can be calculated by superposing the alpha carbon skeleton by the least square method for residues corresponded by the sequence alignment. Furthermore, as an alternative method, it is possible to obtain so called "structure conserved region (SCR)" by repeating the superposition calculations while changing residue correspondence so that the superposition of the steric structures becomes as good as possible.

By displaying the steric structure superposition of multiple kinds of proteins together with the sequence alignment, the relation between the sequence alignment and the steric structures can be easily understood. In the example of Fig.5, a part of the sequence with low similarity between DYR_HUMAN and DYR CHICK (third row to the left of the alignment display) are selected on the screen, and the corresponding parts in the steric structures are highlighted (those parts drawn in bold lines including the upper right alpha helix terminal on the steric structure display).

### Example 5

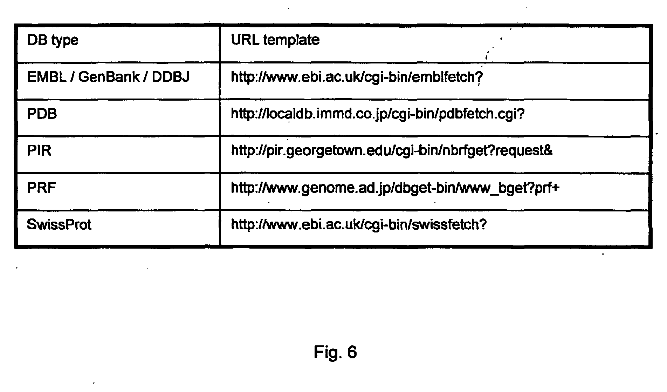

An example of an auxiliary database storing URL templates is shown in Fig. 6. For respective types of external biological sequence information databases, templates of the URL for obtaining the data are stored.

For example, if the data of DYR_HUMAN of the SwissProt are to be obtained from the server computer, it can be carried out by the following procedure. From the record for the SwissProt in the auxiliary database, a template of the URL "http://www.ebi.ac.uk/cgi-bin/swissfetch?" is obtained. By concatenating the ID of the data ("DYR_HUMAN") to be obtained after the character "?" of this template, a URL data "http://www.ebi.ac.uk/cgi-bin/swissfetch?DYR_HUMAN" is generated, and by sending a query to the WWW server using this URL, said data can be downloaded.

### Industrial Applicability

By the present invention, a user can manage and read the information obtained by the search of the biological sequence information easily on the user side terminal such as a personal computer. The user can read the data obtained on the terminal side such as biological sequence information, protein steric structure and sequence alignment, based on the sequence similarity to the data obtained by the user in the past, and further utilize them for the future use by storing them in the user side database.

The present invention is useful when researchers in medicine, pharmaceutical science, agricultural science, molecular biology, genetics, genomics, proteomics and others carry out research using biological sequence information.

## Claims

1. A method of reading biological sequence information which comprises:
(a) a step of designating one or more biological sequence information to be a target of reading as a reading sequence,
(b) a step of judging similarity between the reading sequence and one or more biological sequence information registered in a user side database, and
(c) when the reading sequence is similar to one or more biological sequence information in the user side database, a step of displaying the biological sequence information in the user side database as a similar sequence together with the reading sequence.

2. The method of claim 1, wherein the reading sequence is designated in step (a) based on information displayed on the terminal.

3. The method of claim 2 that uses documents in HTML format, XHTML format, or XML format as the information displayed on the terminal.

4. The method of claim 1, wherein the judgment of similarity in step (b) is carried out by a sequence alignment method.

5. The method of claim 1, wherein the judgment of similarity in step (b) is carried out by the method described in PCT International Publication WO 01/13268 ("Eigen ID").

6. The method of claim 1, wherein a sequence alignment between the reading sequence and the similar sequence is displayed in step (c).

7. The method of claim 1, wherein the relation between the reading sequence and the similar sequence is displayed as a group or with hierarchy in step (c).

8. The method of claim 1, **characterized by** merging and displaying respective annotation information on the reading sequence and the similar sequence in step (c).

9. The method of claim 1, wherein a steric structure of the protein corresponding to either one or both of the reading sequence and the similar sequence is displayed in step (c).

10. The method of claim 1, which further comprises the following step:
(d) a step of storing the reading sequence in the user side database.

11. The method of claim 10, **characterized by** storing the reading sequence and the similar sequence as a group or with hierarchy in step (d).

12. The method of claim 10, wherein respective annotation information on the reading sequence and the similar sequence are merged and stored in step (d).

13. The method of claim 10, which further comprises the following step:
(e) a step of obtaining information from a server computer using a data item in the user side database as a query and setting the information as the reading sequence in step (a).

14. The method of claim 13 which comprises a step of generating an information source specification that corresponds to a data item in the user side database using an auxiliary database storing information sources for respective types of data items.

15. A method of obtaining information from a server computer using a data item in the user side database generated by the method of claim 10 as a query.
